# EUROPÄISCHE PATENTSCHRIFT

(11) **EP 0 529 348 B1**
(45) Veröffentlichungstag und Bekanntmachung des Hinweises auf die Patenterteilung: **23.10.2002**
(21) Anmeldenummer: 92113176.9
(22) Anmeldetag: 03.08.1992
(51) Int. Cl.: C07K 16/18, C12N 5/20, B01D 15/08

(54) **Verfahren zur Reinigung von Faktor XIII, monoklonale Antikörper gegen Faktor XIIIA, ihre Herstellung und Verwendung**
Process for the purification of factor XIII, monoclonal antibodies against factor XIIIA, preparation and use thereof
Procédé de purification du facteur XIII, anticorps monoclonaux contre le facteur XIIIA, leur préparation et utilisation

(30) Priorität: 22.08.1991 DE 4127841
(43) Veröffentlichungstag der Anmeldung: 03.03.1993
(73) Patentinhaber: Aventis Behring Gesellschaft mit beschränkter Haftung, 35002 Marburg (DE)
(72) Erfinder: Hock, Johann, W-3550 Marburg (DE)

(56) Entgegenhaltungen:
- CHEMICAL ABSTRACTS, Band 108, Nr. 13, 28. März 1988, Columbus, Ohio, USA LORAND, L. et al. "Auto-immune antibody (IgG Kansas) against the fibrin stabilizing factor (factor XIII) system." Seite 459, Spalte 2, Zusammenfassung-Nr. 110 500u & Proc. Natl. Acad. Sci. USA 1988, 85(1), 232-236
- CHEMICAL ABSTRACTS, Band 111, Nr. 7, 14. August 1989, Columbus, Ohio, USA IKEMATSU, MASAJIRO et al. "Quantitative determination of blood coagulation factor XIII a2b2 by ELISA." Seite 394, Spalte 1, Zusammenfassung-Nr. 53 777a & JP-A-63 184 061 (TEIKOKU HORMONE) 29. Juli 1988
- CHEMICAL ABSTRACTS, Band 112, Nr. 15, 9. April 1990, Columbus, Ohio, USA TAJIMA, YOSHITAKA et al. "Purification of blood coagulation factor VIII by affinity chromatography using monoclonal antibody." Seite 386, Spalte 1, Zusammenfassung-Nr. 135 586x & JP-A-01 144 991 (CHEMO-SERO-THERAPEUTIC RESEARCH INSTITUTE) 7. Juni 1989
- CHEMICAL ABSTRACTS, Band 112, Nr. 17, 23. April 1990, Columbus, Ohio, USA TAKECHI, KAZUO et al. "Immunoaffinity chromatography of blood coagulation factor IX." Seite 382, Spalte 1, Zusammenfassung-Nr. 154 828u & JP-A-01 258 700 (GREEN CROSS CORP.) 16. Oktober 1989
- CHEMICAL ABSTRACTS, Band 103, Nr. 1, 8. Juli 1990, Columbus, Ohio, USA CHURCH, WILLIAM R. et al. "A simple purification of human factor X using a high affinity monoclonal antibody immunoadsorbent." Seite 297, Spalte 2, Zusammenfassung-Nr. 3 195y & Thromb. Res. 1985, 38(4), 417-424

## Beschreibung

Die Erfindung betrifft ein Verfahren zur Reinigung von Faktor XIII oder XIIIA durch Immunaffinitätschromatographie, monoklonale Antikörper der Klasse IgG gegen Faktor XIIIA, ihre Herstellung und ihre Verwendung.

Faktor XIII ist eine Transglutaminase, die das in der letzten Phase der Blutgerinnung gebildete Fibrin durch die Bildung kovalenter Verbindungen zwischen Fibrinmonomeren stabilisiert. Faktor XIII wird unter anderem im Blutplasma und in Thrombozyten gefunden. Im Plasma tritt er als Komplex von A- und B-Untereinheiten auf, die nicht kovalent miteinander verbunden sind. In Thrombozyten liegt nur die freie A-Untereinheit vor. Für die fibrinstabilisierende Wirkung ist ausschließlich die A-Untereinheit erforderlich. Ein Mangel an Faktor XIIIA (kongenital oder erworben) führt zu ernsten Gerinnungsstörungen, die durch Substitution mit Faktor XIIIA behandelt werden können.

Für die Reinigung von Faktor XIIIA aus verschiedenen Ausgangsmaterialien sind mehrere Verfahren bekannt, die jedoch mit gewissen Nachteilen behaftet sind, wie unzureichender Reinheit des gereinigten Faktor XIIIA, viele Reinigungsschritte und niedrige Ausbeuten.

Bei der Reinigung von Proteinen aus humanen Geweben oder Körperflüssigkeiten für therapeutische Zwecke bringt eine hohe Reinheit ein geringeres Übertragungsrisiko für Viruspartikel mit sich. Bei der Reinigung rekombinanter Proteine aus Fremdorganismen ist eine sehr hohe Reinheit unerläßlich, um antigen wirkende Proteine der Wirtszelle weitestgehend zu entfernen. Die Reinigung von Faktor XIII durch Affinitätschromatographie mit monoklonalen Antikörpern ist daher eine Methode, die sowohl zu einer verbesserten Virussicherheit (bei Reinigung aus menschlichen Geweben oder Körperflüssigkeiten) führt, als auch das Erreichen der erforderlichen Reinheit rekombinanter Produkte erleichtert.

Die Reinigung mit Immunaffinitätschromatographie ist für verschiedene Proteine beschrieben. Für die Reinigung von Enzymen oder anderen biologisch aktiven Molekülen mit Hilfe der Immunaffinitätschromatographie ist jedoch nicht jeder monoklonale Antikörper geeignet, da die Dissoziation des Antigen-Antikörper-Komplexes häufig Bedingungen erfordert, unter denen die biologische Aktivität des Antigens irreversibel zerstört wird (z.B. extreme pH-Werte oder hohe Konzentrationen an chaotropen Salzen). Auch die Reinigung von Faktor XIIIA durch Immunaffinitätschromatographie wurde bereits beschrieben (Gniewek,R.A. et al. (1985) Fed. Proc. 44:1070), wobei Antikörper gegen die B-Untereinheit an einem Träger immobilisiert wurden und der Komplex aus Faktor XIIIA und Faktor XIIIB aus Plasma adsorbiert wurde. Faktor XIIIA wurde dann von der B-Untereinheit dissoziiert und so vom Affinitätsgel eluiert. Mit diesem Verfahren kann Faktor XIIIA aus Human-Placenta oder aus rekombinanten Zellen, die jeweils nur den freien Faktor XIIIA enthalten, nicht gereinigt werden.

Aufgabe der Erfindung ist daher die Entwicklung eines Verfahrens für die immunaffinitätschromatographische Reinigung von Faktor XIII bzw. XIIIA aus Ausgangsmaterialien wie Plasma, Placenta bzw. Extrakten oder Kulturüberständen von rekombinanten Zellen, welche die genetische Information für die Faktor XIII A-Kette enthalten.

Überraschenderweise wurden monoklonale Antikörper der Klasse IgG gegen Faktor XIIIA gefunden, die für die Reinigung von Faktor XIII oder XIIIA aus den genannten Ausgangsmaterialien geeignet sind.

Gegenstand der Erfindung ist somit ein Verfahren zur Reinigung von Faktor XIII oder XIIIA mit Hilfe von Immunaffinitätschromatographie, dadurch gekennzeichnet, daß eine Lösung, die Faktor XIII oder XIIIA enthält, mit einem an ein Trägermaterial gebundenen monoklonalen Antikörper der Klasse IgG gegen Faktor XIIIA (Affinitätsmaterial) in Kontakt gebracht, Affinitätsmaterial und Flüssigkeit voneinander getrennt und Faktor XIII oder XIIIA vom Affinitätsmaterial in biologisch aktiver Form eluiert wird.

Monoklonale Antikörper im Sinne der Erfindung sind auch die dem Fachmann an sich bekannten immunreaktiven Fragmente monoklonaler Antikörper, wie z. B· F_{(ab')2}- , F_{ab}- oder Fᵥ-Fragmente oder antigen-bindende Einzelketten von Antikörper-Molekülen oder Derivate von solchen.

Die in der Literatur beschriebenen monoklonalen Antikörper gegen Faktor XIIIA gehören der Klasse IgM an (Lynch, G. et al. (1985) Thromb. Haemost. 54:274). IgM-Antikörper sind für immunchemische Verfahren im allgemeinen nicht so gut geeignet wie IgG-Antikörper, da sie häufig eine niedrigere Spezifität und Affinität aufweisen.

Aufgabe der Erfindung ist daher außerdem die Herstellung monoklonaler Antikörper der Klasse IgG, gegen Faktor XIIIA.

Durch mehrmalige Immunisierung von Mäusen mit gereinigtem Faktor XIIIA im Abstand von jeweils vier Wochen wurden monoklonale Antikörper gegen Faktor XIIIA der Klasse IgG erhalten.

Gegenstand der Erfindung sind somit auch monoklonale Antikörper der Klasse IgG gegen Faktor XIIIA.

Monoklonale Antikörper können nach Köhler und Milstein (Nature 256:285-308) oder einer der vielen Varianten ihrer Methode (z.B. Goding, J.W. (1980), J.Immunol.Meth. 39:285-308), die dem Fachmann an sich bekannt sind, hergestellt werden, beispielsweise auf folgende Weise:

Säugetiere, bevorzugt Mäuse oder Ratten, werden durch mehrere Injektionen im Abstand von jeweils 1 - 8 Wochen, bevorzugt 4 - 6 Wochen, mit einer Faktor XIII enthaltenden Flüssigkeit, bevorzugt einer Emulsion von gereinigtem Faktor XIIIA in Freund's Adjuvans, immunisiert. Zur Vorbereitung für eine Zellfusion wird Faktor XIIIA 3 - 5 Tage vor dem geplanten Fusionstermin in wässriger Lösung appliziert, bevorzugt intraperitoneal oder intravenös. Zur Gewinnung antikörper-produzierender Zellen wird ein immunisiertes Tier getötet, ein lymphatisches Organ, vorzugsweise die Milz, entnommen und die Lymphocyten freigesetzt. Um in Zellkultur permanent wachsende antikörper-produzierende Zellen zu erhalten, müssen die Lymphozyten immortalisiert werden. Dies kann auf verschiedene Weisen erfolgen, z.B. durch Transformation mit Epstein-Barr-Virus oder Retroviren. Bevorzugt werden jedoch die Lymphozyten mit Myelomzellen fusioniert. Besonders geeignet sind Myelomzellinien, die keine Immunglobuline sezernieren, beispielsweise die Zellinien SP2/0-Ag14 oder X63-Ag8.653. Die Zellen können durch Inkubation mit Polyethylenglykol (PEG) mit einem Molekulargewicht von 1000 - 6000 in 30 - 60 %iger Lösung fusioniert werden, aber auch andere Verfahren, wie z.B. Elektrofusion, sind geeignet. Durch Kultivierung in einem geeigneten Nährmedium werden Hybride aus Lymphozyten und Myelomzellen (Hybridome) selektiert und vermehrt.

Die Hybridome werden 1 - 3 Wochen nach der Zellfusion auf Produktion spezifischer Antikörper getestet. Hierfür steht eine Vielzahl von Testsystemen zur Verfügung, die dem Fachmann an sich bekannt sind. Bevorzugt wird ein ELISA-Testsystem verwendet, bei dem Faktor XIII an eine Festphase adsorbiert ist. Die Zellüberstände der Hybridome werden zunächst mit dem Faktor XIII an der Festphase in Kontakt gebracht, anschließend werden Faktor XIII- spezifische Antikörper durch Inkubation mit einem enzym-markierten Antikörper gegen Maus IgG und nachfolgenden Zusatz eines chromogenen Substrats für das Markierungsenzym detektiert. Zellen, die spezifische Antikörper gegen Faktor XIIIA produzieren, werden durch Vereinzeln unter mikroskopischer Kontrolle oder durch das Verfahren der limitierenden Verdünnung kloniert. Klonale Zellinien werden für die Antikörpergewinnnung in vitro vermehrt. Monoklonale Antikörper werden aus dem verbrauchten Kulturmedium der Zellen gewonnen. Hierzu steht eine Vielzahl von Verfahren zur Verfügung. Vorzugsweise wird
eine Affinitätschromatographie an immobilisiertem Protein A oder Faktor XIIIA durchgeführt.

Die gereinigten monoklonalen Antikörper werden dann auf ihre Eignung für die Immunaffinitätschromatographie getestet. Hierzu werden die einzelnen monoklonalen Antikörper an geeignete Trägermaterialien nach dem Fachmann vertrauten Verfahren gekoppelt. Als Träger finden in der Affinitätschromatographie verschiedene Materialien Verwendung, z.B. Derivate von Agarose, Polyacrylamid oder Cellulose. Die Antikörper können nach Aktivierung des Trägers z.B. mit Bromcyan, Carbodiimid oder durch Oxidation von benachbarten Hydroxylgruppen mit Periodat gekoppelt werden. Eine Vielzahl weiterer Trägermaterialien und Kopplungsmethoden sind dem Fachmann bekannt. Grundsätzlich sind jedes gebräuchliche Trägermaterial und jede Kopplungsmethode geeignet, die die Aktivität der gekoppelten Antikörper nicht oder nur geringfügig beeinträchtigen.

Die Affinitätsgele mit den verschiedenen monoklonalen Antikörpern werden dann auf ihre Eignung für die Reinigung von Faktor XIIIA getestet. Hierzu wird eine Faktor XIIIA enthaltende Lösung über das Affinitätsgel gepumpt und die Faktor XIII-Aktivität im Durchlauf bestimmt. Gele, an die Faktor XIIIA adsorbiert wird, werden auf die Möglichkeit der nativen Elution von Faktor XIIIA geprüft. In einer bevorzugten Verfahrensweise erfolgt die Bindung von Faktor XIIIA an die Immunaffinitätsgele in einem Puffer mit niedriger Ionenstärke, bei einem pH-Wert zwischen 5.5 und 8.5 und bei einer Temperatur von 4° C bis 37° C. Besonders bevorzugt enthält der Puffer 0.01 - 0.05 mol/l Tris oder Phosphat und 0 - 0.1 mol/l NaCl. Die anschließende Dissoziation des Antigen-Antikörper-Komplexes zur Gewinnung von gereinigtem Faktor XIIIA erfordert häufig Bedingungen, unter denen die Aktivität von Faktor XIIIA irreversibel zerstört wird. Es werden daher Antikörper benötigt, die eine Dissoziation des Antigen-Antikörper-Komplexes unter Erhaltung der Faktor XIII-Aktivität erlauben. Grundsätzlich können verschiedene schonende Elutionsmittel verwendet werden, z.B. bestimmte organische Lösungsmittel, Detergentien, wässrige Lösungen von hoher Ionenstärke oder Kombinationen verschiedener Elutionsmittel. Bevorzugt werden für die Elution von Faktor XIIIA hohe Konzentrationen eines Alkali- oder Erdalkalisalzes verwendet, z.B. 0.5 - 3 mol/l NaCl in einem Puffer mit 0.01 - 0.05 mol/l Tris oder Phosphat bei einem pH-Wert von 5.5 - 8.5.

Die folgenden Beispiele erläutern die Erfindung.

### Beispiel 1

### Herstellung des Immunisierungsantigens (gereinigter Faktor XIIIA)

### a) Herstellung eines Immunaffinitätsgels zur Reinigung von Faktor XIIIA

Polyklonale Antikörper gegen Faktor XIIIA wurden aus Antiserum gegen Faktor XIIIA (Behringwerke) durch Affinitätschromatographie gereinigt. Hierzu wurden 10 ml Antiserum über 50 ml Protein A-^{R}Sepharose (Pharmacia) gepumpt. Das Gel wurde mit 500 ml 0.14 mol/l Na₂HPO₄, pH 8.0 gewaschen. Gebundene Antikörper wurden mit 100 ml 0.1 mol/l Glycin, pH 3.0 eluiert und mit 1 mol/l Tris, pH 8.0 auf pH 6.5 eingestellt. Die Antikörper wurden gegen 0.1 mol/l tri-Natrium-Citrat, pH 6.5 (Kopplungspuffer) dialysiert und an Bromcyan-aktivierte ^{R}Sepharose 4B gekoppelt: 15 g Bromcyan-aktivierte ^{R}Sepharose 4B wurden in 1 mmol/l HCl suspendiert, in eine chromatographie-Säule gefüllt und mit 1000 ml HCl (1 mmol/l) gewaschen. Anschließend wurde die Säule mit 50 ml Kopplungspuffer äquilibriert, das Gel in ein verschließbares Gefäß mit 40 ml der gereinigten Antikörper (2 mg/ml in Kopplungspuffer) überführt und zwei Stunden bei Raumtemperatur geschüttelt. Danach wurde das Gel erneut in die Säule gegeben, mit 100 ml Kopplungspuffer gewaschen, in ein verschließbares Gefäß mit 100 ml Ethanolamin-HCl (1 mol/l, pH 8.0) gegeben und zwei Stunden bei Raumtemperatur geschüttelt. Das Gel wurde wieder in die Säule überführt und abwechselnd mit je 300 ml 0.1 mol/l Natriumacetat, 1 mol/l NaCl, pH 4.0 bzw. 0.1 mol/l Tris, 1 mol/l NaCl, pH 8.0 gespült. Der Vorgang wurde 5 mal wiederholt. Zuletzt wurde das Gel mit 0.01 mol/l Na₂HPO₄, 0.01 mol/l NaH₂PO₄, 0.15 mol/l NaCl, pH 7.2 (PBS) äquilibriert und war dann gebrauchsfertig.

### b) Reinigung von Faktor XIIIA

2500 Einheiten von placentärem Faktor XIII (^{R}Fibrogammin, Behringwerke AG) wurden in aqua dest. gelöst und über das Immunaffinitätsgel gepumpt. Das Gel wurde mit PBS gewaschen, und nachdem die Extinktion bei 280 nm kleiner als 0.02 war, mit 0.2 M Glycin, pH 2.5 eluiert. Das Eluat enthielt Faktor XIIIA, der in der SDS-Polyacrylamid-Gelelektrophorese als einzelne Bande erschien.

### Beispiel 2

### Herstellung monoklonaler Antikörper der Klasse IgG gegen Faktor XIIIA

### a) Immunisierung von Mäusen

Weibliche BALB/c-Mäuse wurden durch subkutane Injektion einer Emulsion von 50 µg Faktor XIIIA (hergestellt nach Beispiel 1) in komplettem Freund'schem Adjuvans immunisiert (Tag 1). An den Tagen 28 und 56 wurden je 30 µg Faktor XIIIA, emulgiert in inkomplettem Freund'schem Adjuvans ebenfalls subkutan injiziert. Am Tag 92 folgte eine intraperitoneale Injektion von 100 µg Faktor XIIIA in 0.5 ml physiologischer Kochsalzlösung.

### b) Fusion von Lymphozyten mit Myelomzellen

Am Tag 95 wurden nach Entnahme der Milz durch mechanische Desintegration Lymphozyten gewonnen (ca. 1 x 10⁸ Zellen). Die Lymphozyten wurden in Dulbecco's Modified Eagle's Medium (DMEM) gewaschen, mit 5 x 10⁷ Zellen der Myelomzellinie SP2/0-Ag14 gemischt und abzentrifugiert. Nach vollständiger Entfernung des Überstands wurden dem Zellpellet 0.5 ml einer 50%igen Lösung von Polyethylenglykol 4000 in DMEM innerhalb einer Minute zugetropft. Die Suspension wurde 90 Sekunden bei 37° C inkubiert und anschließend durch Zugabe von 7.5 ml DMEM über einen Zeitraum von 5 Minuten verdünnt. Nach einer Inkubation von 10 Minuten bei Raumtemperatur wurde mit DMEM auf 40 ml aufgefüllt, und die Zellen wurden abzentrifugiert. Nach Absaugen des Überstands wurden die Zellen in DMEM mit 20 % fetalem Kälberserum (FKS) und 13.6 mg/ml Hypoxanthin, 0.18 mg/ml Aminopterin, 3.9 mg/ml Thymidin (HAT-Medium) resuspendiert und auf 6 Mikrotiterplatten ausgesät (200 µl pro Kavität). Verbrauchtes Medium wurde in Abständen von 3 - 4 Tagen durch frisches ersetzt, nach 10 Tagen wurde HAT-Medium durch HT-Medium ersetzt.

### c) Test auf Antikörper gegen Faktor XIIIA

14 Tage nach der Fusion wurden die Zellkulturüberstände der fusionierten Zellen mittels Enzymimmunoassay auf Antikörper gegen Faktor XIIIA getestet: Polystyrol-Mikrotestplatten wurden mit 0.5 µg/ml Faktor XIIIA in 0.1 mol/l NaCl, 0.1 mol/l Natriumacetat, pH 5.5, inkubiert (24 h, 4° C). Anschließend wurden Zellkulturüberstände appliziert (2 h, 37° C), gefolgt von einer Inkubation mit einem peroxidasekonjugierten Antikörper gegen Maus-IgG. Als Substrat wurde eine Lösung von 0.1 % (Gew./Vol.) 2,2'-Azinobis-(3-ethyl-benzothiazolin-6-Sulfonsäure) und 0.012 % (Vol./Vol.) H₂O₂ in 0.1 mol/l Citronensäure, 0.1 mol/l Na₂HPO₄, pH 4.5, verwendet. Nach einer Inkubation von 30 min bei 37° C wurde die Absorption bei 405 nm gemessen. Zwischen den einzelnen Inkubationsschritten wurden die Vertiefungen der Testplatten mit PBS/Tween gewaschen.

### d) Klonierung von Antikörper-produzierenden Zellinien

Zellen, deren Überstände im beschriebenen Enzymimmunoassay eine stark positive Reaktion (Absorption > 1.5) zeigten, wurden nach der Methode der limitierenden Verdünnung kloniert. Dazu wurden ca. 60 Zellen in DMEM mit 20 % FKS und 5 % ^{R}HECS (Costar) auf die 96 Kavitäten einer Zellkulturplatte verteilt. Einzelklone wurden mikroskopisch identifiziert und auf Antikörperproduktion getestet. Die Klonierung wurde zweimal wiederholt.

### e) Reinigung von monoklonalen Antikörpern

Klonale Zellinien wurden zur Produktion von Antikörpern in Roller-Flaschen überführt und in Iscove's Modified Dulbecco's Medium kultiviert. Zellen wurden durch Zentrifugation und Filtration über Papierfilter abgetrennt und mit Hilfe von Ultrafiltration etwa 10fach konzentriert. Das Konzentrat wurde über Protein A-^{R}Sepharose CL-4B (Pharmacia) gegeben, gebundenes IgG wurde mit 0.2 mol/l Glycin/HCl, pH 3.0 eluiert. Die proteinhaltigen Fraktionen wurden gegen 0.1 mol/l Citrat, pH 6.5, dialysiert und mit Ultrafiltration auf ca. 5 mg/ml konzentriert.

### Beispiel 3

### Reinigung von placentärem Faktor XIIIA mittels Immunaffinitätschromatographie

Lyophilisierte Konzentrate von placentärem Faktor XIII (^{R}Fibrogammin, Behringwerke AG) wurden in aqua dest. gelöst. Jeweils 10 ml der Lösung (entsprechend 600 Einheiten Faktor XIII mit einer spezifischen Aktivität von 5.2 E/mg) wurden über ein Affinitätsgel mit einem monoklonalen Antikörper (nach Beispiel 2 gereinigt und nach Beispiel 1 an Bromcyan-aktivierte ^{R}Sepharose 4B gekoppelt) gepumpt. Anschließend wurde nicht gebundenes Protein mit 100 ml Waschpuffer (0.03 mol/l NaCl, 0.02 mol/l Na₂HPO₄, pH 7.4) von der Säule gespült. Faktor XIIIA wurde mit Waschpuffer, der 1 mol/l NaCl enthielt, eluiert. Die spezifische Aktivität betrug 90 - 105 E/mg; das Protein erschien in der SDS-Polyacrylamid-Gelelektrophorese als eine Bande mit einem Molekulargewicht von 75.000 Dalton.

## Patentansprüche (Patentansprüche für folgende(n) Vertragsstaat(en): AT, IE)

1. Verfahren zur Reinigung des Gerinnungsfaktors XIII oder XIIIA mittels Immunaffinitätschromatographie, **dadurch gekennzeichnet, daß** eine Lösung, die Faktor XIII oder XIIIA enthält, mit einem an ein Trägermaterial gebundenen monoklonalen Antikörper der Klasse IgG gegen Faktor XIIIA (Affinitätsmaterial) in Kontakt gebracht, Affinitätsmaterial und Flüssigkeit voneinander getrennt und Faktor XIII beziehungsweise XIIIA vom Affinitätsmaterial in biologisch aktiver Form eluiert wird.

2. Verfahren nach Anspruch 1, **dadurch gekennzeichnet, daß** ein immunreaktives Fragment oder Derivat eines monoklonalen Antikörpers der Klasse IgG an das Trägermaterial gebunden ist.

3. Verfahren nach Anspruch 1 oder 2, **dadurch gekennzeichnet, daß** mit einem Puffer eluiert wird, der 0.5 - 3 mol/l eines Alkali- oder Erdalkalisalzes enthält.

4. Monoklonaler Antikörper der Klasse IgG gegen Faktor XIIIA, wobei dieser Antikörper die Elution des gebunden F XIII a in biologisch aktiver Form ermöglicht.

5. Monoklonaler Antikörper nach Anspruch 4 gebunden an ein Trägermaterial.

6. Verwendung eines monoklonalen Antikörpers der Klasse IgG gegen Faktor XIIIA oder eines Fragments oder Derivates eines solchen Antikörpers zur Reinigung von Faktor XIII oder XIIIA.

## Patentansprüche (Patentansprüche für folgende(n) Vertragsstaat(en): ES, GR)

1. Verfahren zur Reinigung des Gerinnungsfaktors XIII oder XIIIA mittels Immunaffinitätschromatographie, **dadurch gekennzeichnet, daß** eine Lösung, die Faktor XIII oder XIIIA enthält, mit einem an ein Trägermaterial gebundenen monoklonalen Antikörper der Klasse IgG gegen Faktor XIIIA (Affinitätsmaterial) in Kontakt gebracht, Affinitätsmaterial und Flüssigkeit voneinander getrennt und Faktor XIII beziehungsweise XIIIA vom Affinitätsmaterial in biologisch aktiver Form eluiert wird.

2. Verfahren nach Anspruch 1, **dadurch gekennzeichnet, daß** ein immunreaktives Fragment oder Derivat eines monoklonalen Antikörpers der Klasse IgG an das Trägermaterial gebunden ist.

3. Verfahren nach Anspruch 1 oder 2, **dadurch gekennzeichnet, daß** mit einem Puffer eluiert wird, der 0.5 - 3 mol/l eines Alkali- oder Erdalkalisalzes enthält.

## Claims (Claims for the following Contracting State(s): AT, IE)

1. A process for the purification of coagulation factor XIII or XIIIA by immunoaffinity chromatography, which comprises a solution which contains factor XIII or XIIIA being contacted with a monoclonal antibody of the IgG class against factor XIIIA which is bound to a support material (affinity material), separating the affinity material and liquid from one another, and eluting factor XIII or XIIIA from the affinity material in biologically active form.

2. The process as claimed in claim 1, wherein an immunoreactive fragment or derivative of a monoclonal antibody of the IgG class is bound to the support material.

3. The process as claimed in claim 1 or 2, wherein a buffer which contains 0.5 - 3 mol/l of an alkali metal or alkaline earth metal salt is used for elution.

4. A monoclonal antibody of the IgG class against factor XIIIA, the antibody making it posssible to elute the bound F XIII a in biologically active form.

5. A monoclonal antibody as claimed in claim 4 bound to a support material.

6. The use of a monoclonal antibody of the IgG class against factor XIIIA or of a fragment or derivative of an antibody of this type for purifying factor XIII or XIIIA.

## Claims (Claims for the following Contracting State(s): ES, GR)

1. A process for the purification of coagulation factor XIII or XIIIA by immunoaffinity chromatography, which comprises a solution which contains factor XIII or XIIIA being contacted with a monoclonal antibody of the IgG class against factor XIIIA which is bound to a support material (affinity material), separating the affinity material and liquid from one another, and eluting factor XIII or XIIIA from the affinity material in biologically active form.

2. The process as claimed in claim 1, wherein an immunoreactive fragment or derivative of a monoclonal antibody of the IgG class is bound to the support material.

3. The process as claimed in claim 1 or 2, wherein a buffer which contains 0.5 - 3 mol/l of an alkali metal or alkaline earth metal salt is used for elution.

## Revendications (Revendications pour l'(les) Etat(s) contractant(s) suivant(s): AT, IE)

1. Procédé pour la purification du facteur XIII ou XIIIA à l'aide de la chromatographie d'immunoaffinité, **caractérisé en ce qu'**on met une solution, qui contient du facteur XIII ou XIIIA, en contact avec un anticorps monoclonal de la classe IgG, dirigé contre le facteur XIIIA, fixé sur un matériau de support, (matériau d'affinité), on sépare l'un de l'autre le matériau d'affinité et le liquide, et le facteur XIII ou XIIIA est élué sous forme biologiquement active du matériau d'affinité.

2. Procédé selon la revendication 1, **caractérisé en ce qu'**un fragment ou dérivé immunoréactif d'un anticorps monoclonal de la classe IgG est fixé au matériau de support.

3. Procédé selon la revendication 1 ou 2, **caractérisé en ce qu'**on effectue l'élution avec un tampon qui contient 0,5-3 moles/l d'un sel alcalin ou alcalino-terreux.

4. Anticorps monoclonal de la classe IgG contre le facteur XIIIA, cet anticorps permettant l'élution du F XIII a fixé, sous une forme biologiquement active.

5. Anticorps monoclonal selon la revendication 4, fixé à un matériau de support.

6. Utilisation d'un anticorps monoclonal de la classe IgG anti-facteur XIIIA ou d'un fragment ou dérivé d'un tel anticorps, pour la purification du facteur XIII ou XIIIA.

## Revendications (Revendications pour l'(les) Etat(s) contractant(s) suivant(s): ES, GR)

1. Procédé pour la purification du facteur XIII ou XIIIA à l'aide de la chromatographie d'immunoaffinité, **caractérisé en ce qu'**on met une solution, qui contient du facteur XIII ou XIIIA, en contact avec un anticorps monoclonal de la classe IgG, dirigé contre le facteur XIIIA, fixé sur un matériau de support, (matériau d'affinité), on sépare l'un de l'autre le matériau d'affinité et le liquide, et le facteur XIII ou XIIIA est élué sous forme biologiquement active du matériau d'affinité.

2. Procédé selon la revendication 1, **caractérisé en ce qu'**un fragment ou dérivé immunoréactif d'un anticorps monoclonal de la classe IgG est fixé au matériau de support.

3. Procédé selon la revendication 1 ou 2, **caractérisé en ce qu'**on effectue l'élution avec un tampon qui contient 0,5-3 moles/l d'un sel alcalin ou alcalino-terreux.
